# EUROPEAN PATENT APPLICATION

(11) **EP 3 771 433 A1**
(43) Date of publication of application: **03.02.2021**
(21) Application number: 19189159.7
(22) Date of filing: 30.07.2019
(51) Int. Cl.: A61B 8/08, A61B 8/00

(54) **DIFFERENTIATING PASSIVE ULTRASOUND SENSORS FOR INTERVENTIONAL MEDICAL PROCEDURES**

(71) Applicant: Koninklijke Philips N.V., 5656 AG Eindhoven (NL)
(72) Inventor: ERKAMP, Ramon Quido, 5656 AE Eindhoven (NL); CHEN, Alvin, 5656 AE Eindhoven (NL); BHARAT, Shyam, 5656 AE Eindhoven (NL); VAIDYA, Kunal, 5656 AE Eindhoven (NL); JAIN, Ameet Kumar, 5656 AE Eindhoven (NL); VIGNON, Francois Guy Gerard Marie, 5656 AE Eindhoven (NL)
(74) Representative: de Haan, Poul Erik

(57) **Abstract**

A controller (250) for differentiating passive ultrasound sensors for interventional medical procedures includes a memory (291) and a processor (292). When executed by the processor (292), instructions from the memory (291) cause a system (200) that includes the controller (250) to implement a process that includes receiving first signals from a first passive ultrasound sensor (S1) and receiving second signals from a second passive ultrasound sensor (S2). The first signals and second signals are generated by the passive ultrasound sensors responsive to beams emitted from an ultrasound imaging probe (210). The process also includes identifying a characteristic of the first signals and the second signals. The characteristic includes shapes of the first signals and the second signals and/or times at which the first signals and the second signals are generated as the beams from the ultrasound imaging probe are received. The first passive ultrasound sensor (S1) and the second passive ultrasound sensor (S2) are differentiated based on the characteristic.

## Description

### BACKGROUND OF THE INVENTION

In ultrasound imaging, the visibility of an interventional medical device such as a needle or catheter is often very poor due to the specular nature of the needle surface that reflects beams away from the ultrasound imaging probe. To alleviate this problem some needle manufacturers have produced needles with special echogenic coatings, but the visualization improvement is limited. Ultrasound imaging system manufacturers have developed algorithms that use multiple imaging beams from different angles, but improvements from the algorithms are limited and such a strategy is primarily suited only for linear imaging arrays. Both strategies do not help when the needle is inserted perpendicular to the imaging plane or the needle path has a small offset relative to the imaging plane.

Ultrasound tracking technology estimates the position of a passive ultrasound sensor (e.g., PZT, PVDF, copolymer or other piezoelectric material) in the field of view (FOV) of a diagnostic ultrasound B-mode image by analyzing the signal received by the passive ultrasound sensor as imaging beams from an ultrasound probe sweep the field of view. A passive ultrasound sensor is an acoustic pressure sensor, and these passive ultrasound sensors are used to determine location of an interventional medical device. Time-of-flight measurements provide the axial/radial distance of the passive ultrasound sensor from an imaging array of the ultrasound probe, while amplitude measurements and knowledge of the direct beam firing sequence provide the lateral / angular position of the passive ultrasound sensor.

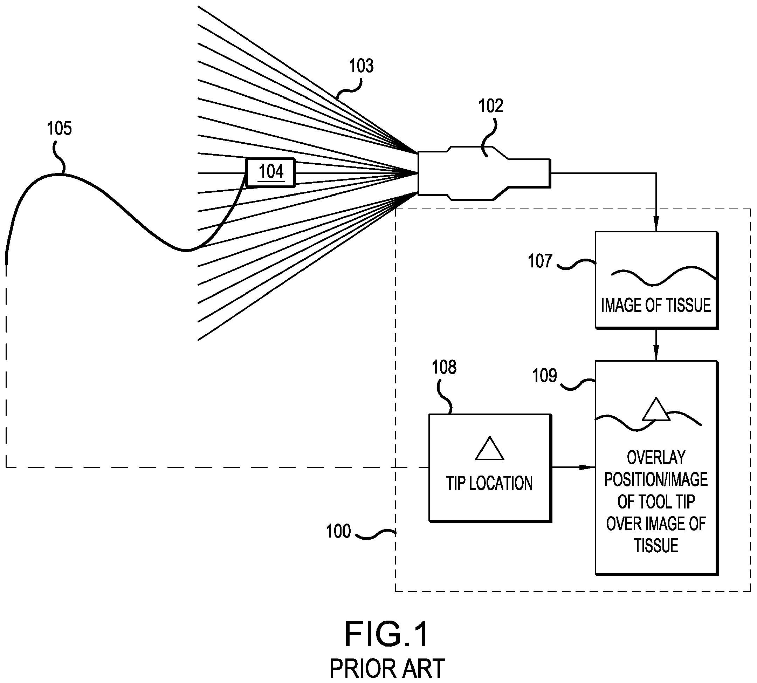

FIG. 1 illustrates a known system for tracking an interventional medical device using a passive ultrasound sensor. The known system in FIG. 1 may be known by the name "Insitu", which stands for Intelligent Sensing of Tracked Instruments using Ultrasound. In FIG. 1, an ultrasound probe 102 emits an imaging beam 103 that sweeps across a passive ultrasound sensor 104 on a tip of an interventional medical device 105. Timing information of the imaging beam 103 being emitted is sent as a line trigger (and/or frame trigger) for a signal processing algorithm to determine a location of the passive ultrasound sensor 104 on the tip of the interventional medical device 105 as a tip location 108. An image of tissue 107 is fed back by the ultrasound probe 102. The location of the passive ultrasound sensor 104 on the tip of the interventional medical device 105 is provided as the tip location 108 upon determination by the signal processing algorithm. The tip location 108 is overlaid on the image of tissue 107 as an overlay image 109. The image of tissue 107, the tip location 108, and the overlay image 109 are all displayed on a display 100. The tip location 108 is calculated with a positional accuracy that may exceed 0.5mm, depending on the type of the ultrasound probe 102, even under conditions where the interventional medical device is not visible in the ultrasound image. Generally, the positional accuracy may be on the same order as the resolution in the image of the tissue. For high frequency linear ultrasound probes imaging at shorter depths the positional accuracy may be better than 0.5mm. For cardiac ultrasound probes imaging at deep depths, the positional accuracy depends on the depth of the passive ultrasound sensor 104 as the imaging beams are not parallel but are instead fan-shaped as shown by imaging beam 103. At deeper depths the beam spacing is wider, and the positional accuracy thus lower.

### SUMMARY OF THE INVENTION

According to an aspect of the present disclosure, a controller for differentiating passive ultrasound sensors for interventional medical procedures includes a memory that stores instructions, and a processor that executes the instructions. When executed by the processor, the instructions cause a system that includes the controller to implement a process that includes receiving first signals from a first passive ultrasound sensor that generates the first signals responsive to beams emitted from an ultrasound imaging probe and receiving second signals from a second passive ultrasound sensor that generates the second signals responsive to the beams emitted from the ultrasound imaging probe. The process implemented by the controller also includes identifying a characteristic of the first signals and the second signals including at least one of shapes of the first signals and the second signals and times at which the first signals and the second signals are generated as the beams from the ultrasound imaging probe are received. The process implemented by the controller further includes differentiating between the first passive ultrasound sensor and the second passive ultrasound sensor based on the characteristic including the at least one of shapes of the first signals and the second signals and the times at which the first signals and the second signals are generated as the beams from the ultrasound imaging probe are received.

According to another aspect of the present disclosure, a tangible non-transitory computer readable storage medium stores a computer program. When executed by a processor, the computer program causes a system that includes the tangible non-transitory computer readable storage medium to perform a process for differentiating passive ultrasound sensors for interventional medical procedures. The process performed when the processor executes the computer program from the tangible non-transitory computer readable storage medium includes receiving first signals from a first passive ultrasound sensor that generates the first signals responsive to beams emitted from an ultrasound imaging probe and receiving second signals from a second passive ultrasound sensor that generates the second signals responsive to the beams emitted from the ultrasound imaging probe. The process implemented when the processor executes the computer program also includes identifying a characteristic of the first signals and the second signals including at least one of shapes of the first signals and the second signals and times at which the first signals and the second signals are generated as the beams from the ultrasound imaging probe are received. The process implemented when the processor executes the computer program further includes differentiating between the first passive ultrasound sensor and the second passive ultrasound sensor based on the characteristic including the at least one of shapes of the first signals and the second signals and the times at which the first signals and the second signals are generated as the beams from the ultrasound imaging probe are received.

According to yet another aspect of the present disclosure, a system for differentiating passive ultrasound sensors for interventional medical procedures includes a first passive ultrasound sensor that generates and sends first signals responsive to beams emitted from an ultrasound imaging probe during an interventional medical procedure, and a second passive ultrasound sensor that generates and sends second signals responsive to the beams emitted from the ultrasound imaging probe. The first signals and the second signals include an identifiable characteristic including at least one of shapes of the first signals and the second signals and times at which the first signals and the second signals are generated as the beams from the ultrasound imaging probe are received. As a result, the first passive ultrasound sensor and the second passive ultrasound sensor can be differentiated based on the characteristic including the at least one of shapes of the first signals and the second signals and the times at which the first signals and the second signals are generated as the beams from the ultrasound imaging probe are received.

### BRIEF DESCRIPTION OF THE DRAWINGS

The example embodiments are best understood from the following detailed description when read with the accompanying drawing figures. It is emphasized that the various features are not necessarily drawn to scale. In fact, the dimensions may be arbitrarily increased or decreased for clarity of discussion. Wherever applicable and practical, like reference numerals refer to like elements.
FIG. 1 illustrates a known system for tracking an interventional medical device using a passive ultrasound sensor.
FIG. 2A illustrates a system for differentiating passive ultrasound sensors for interventional medical procedures, in accordance with a representative embodiment.
FIG. 2B illustrates a controller for differentiating passive ultrasound sensors for interventional medical procedures, in accordance with a representative embodiment.
FIG. 3 illustrates a process for differentiating passive ultrasound sensors for interventional medical procedures, in accordance with a representative embodiment.
FIG. 4 illustrates a set of passive ultrasound sensors with differentiating bias voltages in a single configuration for differentiating passive ultrasound sensors for interventional medical procedures, in accordance with a representative embodiment.
FIG. 5 illustrates another process for differentiating passive ultrasound sensors for interventional medical procedures, in accordance with a representative embodiment.
FIG. 6 illustrates another process for differentiating passive ultrasound sensors for interventional medical procedures, in accordance with a representative embodiment.
FIG. 7 illustrates a set of passive ultrasound sensors with differentiating connections in a single configuration for differentiating passive ultrasound sensors for interventional medical procedures, in accordance with a representative embodiment.
FIG. 8 illustrates another set of passive ultrasound sensors with differentiating connections in a single configuration for differentiating passive ultrasound sensors for interventional medical procedures, in accordance with a representative embodiment.

### DETAILED DESCRIPTION OF EMBODIMENTS

In the following detailed description, for purposes of explanation and not limitation, representative embodiments disclosing specific details are set forth in order to provide a thorough understanding of an embodiment according to the present teachings. Descriptions of known systems, devices, materials, methods of operation and methods of manufacture may be omitted so as to avoid obscuring the description of the representative embodiments. Nonetheless, systems, devices, materials and methods that are within the purview of one of ordinary skill in the art are within the scope of the present teachings and may be used in accordance with the representative embodiments. It is to be understood that the terminology used herein is for purposes of describing particular embodiments only and is not intended to be limiting. The defined terms are in addition to the technical and scientific meanings of the defined terms as commonly understood and accepted in the technical field of the present teachings.

It will be understood that, although the terms first, second, third etc. may be used herein to describe various elements or components, these elements or components should not be limited by these terms. These terms are only used to distinguish one element or component from another element or component. Thus, a first element or component discussed below could be termed a second element or component without departing from the teachings of the inventive concept.

The terminology used herein is for purposes of describing particular embodiments only and is not intended to be limiting. As used in the specification and appended claims, the singular forms of terms 'a', 'an' and 'the' are intended to include both singular and plural forms, unless the context clearly dictates otherwise. Additionally, the terms "comprises", and/or "comprising," and/or similar terms when used in this specification, specify the presence of stated features, elements, and/or components, but do not preclude the presence or addition of one or more other features, elements, components, and/or groups thereof. As used herein, the term "and/or" includes any and all combinations of one or more of the associated listed items.

Unless otherwise noted, when an element or component is said to be "connected to", "coupled to", or "adjacent to" another element or component, it will be understood that the element or component can be directly connected or coupled to the other element or component, or intervening elements or components may be present. That is, these and similar terms encompass cases where one or more intermediate elements or components may be employed to connect two elements or components. However, when an element or component is said to be "directly connected" to another element or component, this encompasses only cases where the two elements or components are connected to each other without any intermediate or intervening elements or components.

In view of the foregoing, the present disclosure, through one or more of its various aspects, embodiments and/or specific features or sub-components, is thus intended to bring out one or more of the advantages as specifically noted below. For purposes of explanation and not limitation, example embodiments disclosing specific details are set forth in order to provide a thorough understanding of an embodiment according to the present teachings. However, other embodiments consistent with the present disclosure that depart from specific details disclosed herein remain within the scope of the appended claims. Moreover, descriptions of well-known apparatuses and methods may be omitted so as to not obscure the description of the example embodiments. Such methods and apparatuses are within the scope of the present disclosure.

As described herein, multiple passive ultrasound sensors can be used to track the position of an interventional medical device such as the device tip, as well as the orientation and/or shape of the interventional medical device.

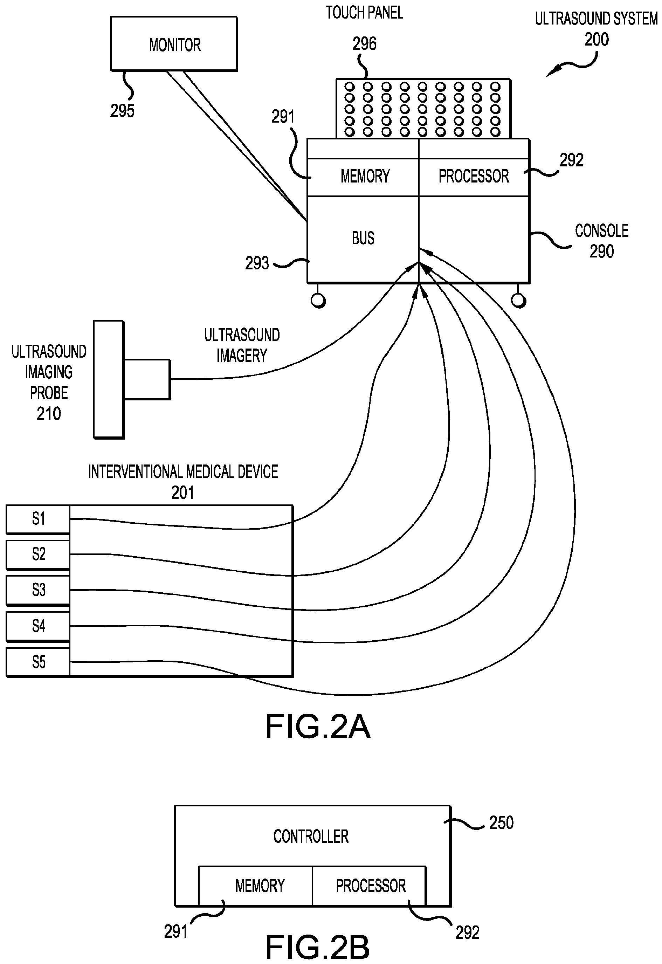

FIG. 2A illustrates a system for differentiating passive ultrasound sensors for interventional medical procedures, in accordance with a representative embodiment.

The ultrasound system 200 in FIG. 2A includes an interventional medical device 201, an ultrasound imaging probe 210, a console 290 and five separate passive ultrasound sensors. The five passive ultrasound sensors in FIG. 2A include a first passive ultrasound sensor S1, a second passive ultrasound sensor S2, a third passive ultrasound sensor S3, a fourth passive ultrasound sensor S4 and a fifth passive ultrasound sensor S5. The console 290 includes a memory 291, a processor 292, a bus 293, a monitor 295 and a touch panel 296.

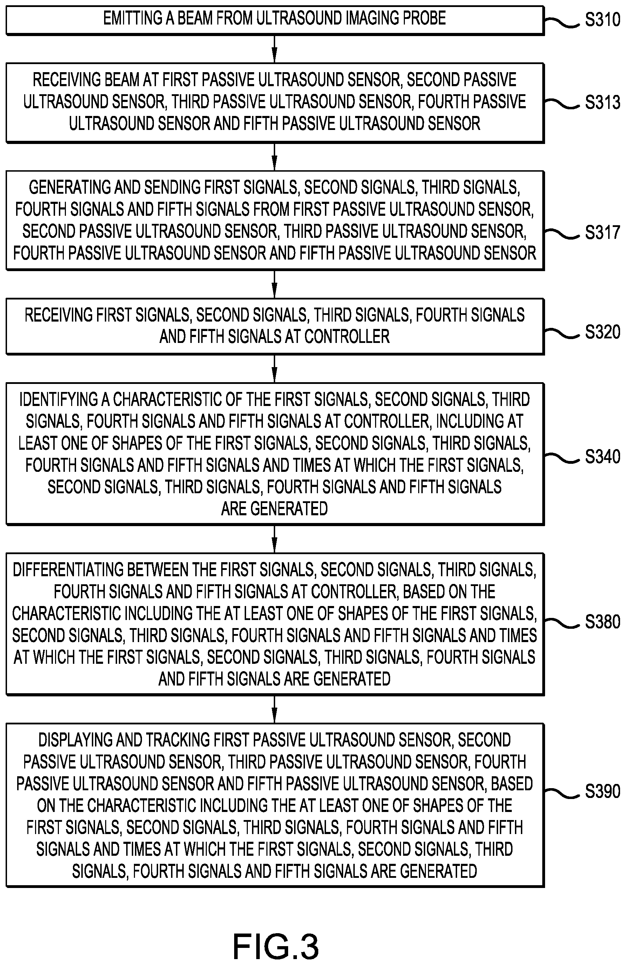

The use of five passive ultrasound sensors in FIG. 2A is for an embodiment described herein but is not necessary for other embodiments. Differentiation as described herein requires at least two passive ultrasound sensors, and in the embodiment of FIG. 3 there are three passive ultrasound sensors. Accordingly, differentiation can be implemented for two or more passive ultrasound sensors consistent with the teachings of embodiments herein.

FIG. 2B illustrates a controller for differentiating passive ultrasound sensors for interventional medical procedures, in accordance with a representative embodiment.

The controller 250 in FIG. 2B includes the memory 291 and the processor 292. Although the controller 250 includes elements of the console 290 from FIG. 2A, a controller 250 may be implemented separate from the console 290 such as by a personal computer or a mobile computer.

A processor 292 for a controller 250 is tangible and non-transitory. As used herein, the term "non-transitory" is to be interpreted not as an eternal characteristic of a state, but as a characteristic of a state that will last for a period. The term "non-transitory" specifically disavows fleeting characteristics such as characteristics of a carrier wave or signal or other forms that exist only transitorily in any place at any time. A processor 292 is an article of manufacture and/or a machine component. A processor 292 for a controller 250 is configured to execute software instructions to perform functions as described in the various embodiments herein. A processor 292 for a controller 250 may be a general-purpose processor or may be part of an application specific integrated circuit (ASIC). A processor 292 for a controller 250 may also be a microprocessor, a microcomputer, a processor chip, a controller, a microcontroller, a digital signal processor (DSP), a state machine, or a programmable logic device. A processor 292 for a controller may also be a logical circuit, including a programmable gate array (PGA) such as a field programmable gate array (FPGA), or another type of circuit that includes discrete gate and/or transistor logic. A processor 292 for a controller 250 may be a central processing unit (CPU), a graphics processing unit (GPU), or both. Additionally, any processor described herein may include multiple processors, parallel processors, or both. Multiple processors may be included in, or coupled to, a single device or multiple devices. A "processor" as used herein encompasses an electronic component which is able to execute a program or machine executable instruction. References to the computing device comprising "a processor" should be interpreted as possibly containing more than one processor or processing core. The processor may for instance be a multi-core processor. A processor may also refer to a collection of processors within a single computer system or distributed amongst multiple computer systems. The term computing device should also be interpreted to possibly refer to a collection or network of computing devices each including a processor or processors. Many programs have instructions performed by multiple processors that may be within the same computing device or which may even be distributed across multiple computing devices.

Memories such as the memory 291 described herein are tangible storage mediums that can store data and executable instructions and are non-transitory during the time instructions are stored therein. As used herein, the term "non-transitory" is to be interpreted not as an eternal characteristic of a state, but as a characteristic of a state that will last for a period. The term "non-transitory" specifically disavows fleeting characteristics such as characteristics of a carrier wave or signal or other forms that exist only transitorily in any place at any time. A memory described herein is an article of manufacture and/or machine component. Memories described herein are computer-readable mediums from which data and executable instructions can be read by a computer. Memories as described herein may be random access memory (RAM), read only memory (ROM), flash memory, electrically programmable read only memory (EPROM), electrically erasable programmable read-only memory (EEPROM), registers, a hard disk, a removable disk, tape, compact disk read only memory (CD-ROM), digital versatile disk (DVD), floppy disk, blu-ray disk, or any other form of storage medium known in the art. Memories may be volatile or non-volatile, secure and/or encrypted, unsecure and/or unencrypted. "Memory" is an example of a computer-readable storage medium. Computer memory is any memory which is directly accessible to a processor. Examples of computer memory include, but are not limited to RAM memory, registers, and register files. References to "computer memory" or "memory" should be interpreted as possibly being multiple memories. The memory may for instance be multiple memories within the same computer system. The memory may also be multiple memories distributed amongst multiple computer systems or computing devices.

FIG. 3 illustrates a process for differentiating passive ultrasound sensors for interventional medical procedures, in accordance with a representative embodiment.

In FIG. 3, the process starts at S310 with emitting a beam from an ultrasound imaging probe. The ultrasound imaging probe used at S310 may be the ultrasound imaging probe 210 in FIG. 2A.

At S313, the process in FIG. 3 continues with receiving the beam at a first passive ultrasound sensor, a second passive ultrasound sensor, a third passive ultrasound sensor, a fourth passive ultrasound sensor, and a fifth passive ultrasound sensor. The five passive ultrasound sensors that receive the beam at S313 may be the first passive ultrasound sensor S1, the second passive ultrasound sensor S2, the third passive ultrasound sensor S3, the fourth passive ultrasound sensor S4 and the fifth passive ultrasound sensor S5 in FIG. 2A. However, consistent with the description of various embodiments herein, fewer than five passive ultrasound sensors may be used while still achieving the differentiation described herein.

At S317, the process in FIG. 3 includes generating and sending first signals, second signals, third signals, fourth signals and fifth signals from the first passive ultrasound sensor, the second passive ultrasound sensor, the third passive ultrasound sensor, the fourth passive ultrasound sensor, and the fifth passive ultrasound sensor. The five passive ultrasound sensors that generate and send the signals at S317 may be the first passive ultrasound sensor S1, the second passive ultrasound sensor S2, the third passive ultrasound sensor S3, the fourth passive ultrasound sensor S4 and the fifth passive ultrasound sensor S5 in FIG. 2A.

At S320, the process in FIG. 3 continues with receiving the first signals, the second signals, the third signals, the fourth signals, and the fifth signals at the controller. The controller that receives the signals at S320 may be the controller 250 in FIG. 2B, whether implemented as part of the console 290 in FIG. 2A or otherwise. Additionally, the signals may be received over separate wired connections at ports on the controller 250 or on or in an interface dedicated to the controller 250.

At S340, the process in FIG. 3 includes identifying a characteristic of the first signals, the second signals, the third signals, the fourth signals and the fifth signals at the controller. The characteristic that is identified includes at least one of shapes of the first signals, the second signals, the third signals, the fourth signals and the fifth signals, and times at which the first signals, the second signals, the third signals, the fourth signals and the fifth signals are generated. The identifying at S340 may be implemented by the controller 250 in FIG. 2B, whether implemented as part of the console 290 in FIG. 2A or otherwise.

At S380, the process in FIG. 3 includes differentiating between the first signals, the second signals, the third signals, the fourth signals and the fifth signals. The differentiating at S380 is based on the characteristic including the at least one of shapes of the first signals, the second signals, the third signals, the fourth signals and the fifth signals, and the times at which the first signals, the second signals, the third signals, the fourth signals and the fifth signals are generated. The differentiating at S380 may be implemented by the controller 250 in FIG. 2B, whether implemented as part of the console 290 in FIG. 2A or otherwise.

At S390, the process in FIG. 3 includes displaying and tracking the first passive ultrasound sensor, the second passive ultrasound sensor, the third passive ultrasound sensor, the fourth passive ultrasound sensor and the fifth passive ultrasound sensor. The displaying and tracking at S390 is also based on the characteristic including the at least one of shapes of the first signals, the second signals, the third signals, the fourth signals and the fifth signals, and the times at which the first signals, the second signals, the third signals, the fourth signals and the fifth signals are generated. The displaying at S390 may be performed using the monitor 295 in FIG. 2A, whereas the tracking at S390 may be performed using a combination of the monitor 295 in FIG. 2A and the controller 250 in FIG. 2B. That is, the passive ultrasound sensors may be tracked by a software program implemented by the controller 250, resulting in a display of the locations of the passive ultrasound sensors on the monitor 295.

As an example embodiment consistent with FIG. 3, a shape of a radio frequency (RF) wave from a passive ultrasound sensor is a function of both the acoustic insonification and the size/geometry of the receiving passive ultrasound sensor. Thus, the shape of the waves of the signals may reflect sizes of the passive ultrasound sensors. For example, if the spatial extent of the passive ultrasound sensor is larger, the received electrical signal will have a longer time duration. Accordingly, the shapes of signals reflect at least one of sizes of passive ultrasound sensors and time durations during which the passive ultrasound sensors received beams emitted from an ultrasound imaging probe 210.

Also, larger passive ultrasound sensors will generally suppress higher frequency components more, so different passive ultrasound sensor geometries can lead to different spectral content of the received signal. When connecting multiple passive ultrasound sensors with significantly differing geometry in parallel, the passive ultrasound sensor responsible for a received electrical signal can be identified by examining the shape of the received signal. That is, different shapes of received radio frequency waves may be specifically correlated to different passive ultrasound sensors of different sizes precisely because of the differences in sizes of the passive ultrasound sensors. Thus, use of shape of a signal as the characteristic in the embodiment in FIG. 3 may result in the differentiation and the displaying and tracking as example practical applications set forth in FIG. 3.

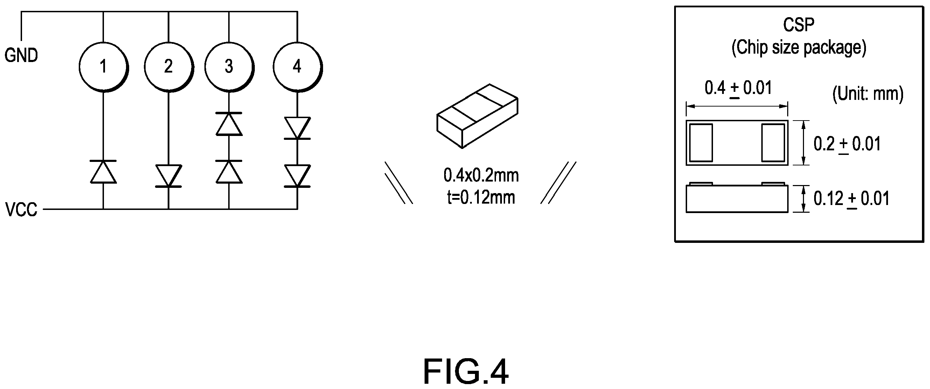

FIG. 4 illustrates a set of passive ultrasound sensors with differentiating bias voltages in a single configuration for differentiating passive ultrasound sensors for interventional medical procedures, in accordance with a representative embodiment.

In FIG. 4, the set of passive ultrasound sensors includes the first passive ultrasound sensor S1 (labelled 1 in a circle), the second passive ultrasound sensor S2 (labelled 2 in a circle), the third passive ultrasound sensor S3 (labelled 3 in a circle), and the fourth passive ultrasound sensor S4 (labelled 4 in a circle). Each of the set of passive ultrasound sensors in FIG. 4 is provided on a separate line between an input voltage (VCC) and ground (GND).

As a general matter, diodes need to have a forward voltage of 0.6V across the diode to become conductive. Additionally, passive ultrasound sensors are often amplified using a charge amplifier, because charge amplifiers keep the voltage across the sensor constant and thereby mitigate the effects of parasitic capacitance on the interconnecting wires. Normally this constant voltage that the charge amplifier generates would be 0V, but this constant direct current (DC) voltage can be switched to any other desirable value. In the embodiment of FIG. 4, the four passive ultrasound sensors are connected in parallel, but with varying diode configurations in series. When the charge amplifier is set to generate, for example, 0.9 volts, only the diode connected to the first passive ultrasound sensor S1 is in a conductive state. When the bias voltage is increased to 1.5 volts, both the first passive ultrasound sensor S1 and the third passive ultrasound sensor S3 are connected. Similarly, when the bias voltage is decreased to -0.9 volts, only the second passive ultrasound sensor S2 is connected. And when the bias voltage is decreased to -1.5 volts, the second passive ultrasound sensor S2 and the fourth passive ultrasound sensor S4 are both connected. Accordingly, a controller may be used to control an amplifier voltage from an amplifier to bias the passive ultrasound sensors with varied bias voltages.

In the embodiment of FIG. 4, an imaging beam can be fired repeatedly, such as four times, in the same direction while cycling through the different bias voltages. Based on the different bias voltages, a determination can be made as to which of the four passive ultrasound sensors is generating the electrical signal that is received. That is, the different bias voltages are correlated with the received electrical signals so that each electrical signal from a passive ultrasound sensor can be correlated going forward with the passive ultrasound sensor, such as during an interventional medical procedure.

In FIG. 4, the bias voltages are an example of system states that can be used to differentiate between different passive ultrasound sensors. Another example of a system state is identification of which leads are serving as the basis of a detected signal.

Thus, use of different bias voltages applied uniformly to amplifiers correlated with different passive ultrasound sensors to generate the signal can be used in the differentiation and the displaying and tracking as example practical applications set forth in FIG. 4. For example, the timing of when the different signals are generated or not generated can be correlated to the different bias voltages that are fired, which in turn can be used to differentiate which signals are from which passive ultrasound sensors.

In FIG. 4, relative dimensions of a chip that includes the diodes and passive ultrasound sensors are shown in millimeters (mm). This is only shown for context, and not to be limiting. For example, a chip that includes one or more diodes and one or more passive ultrasound sensors may have a width of .4mm, a depth of .2mm and a height of .12mm, with close tolerances in each dimension of +/-.01mm.

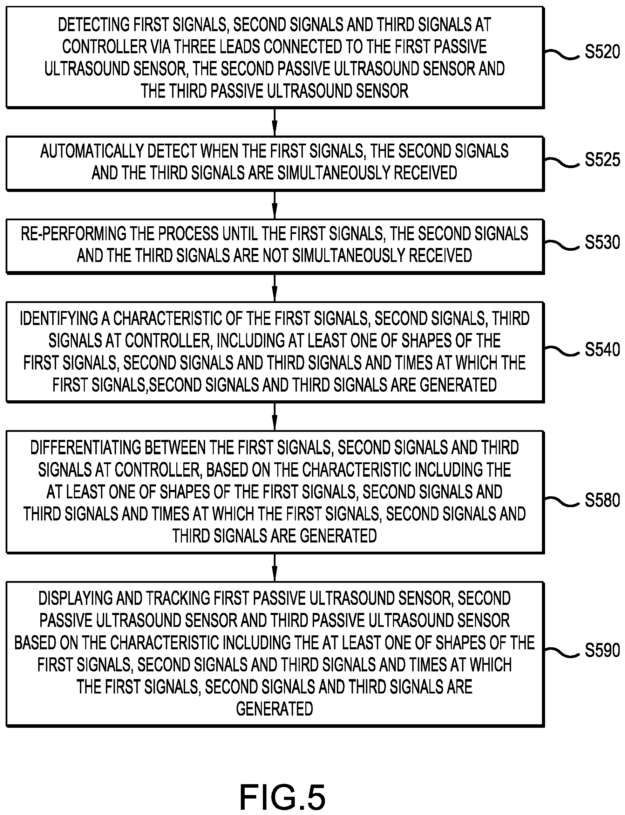

FIG. 5 illustrates another process for differentiating passive ultrasound sensors for interventional medical procedures, in accordance with a representative embodiment.

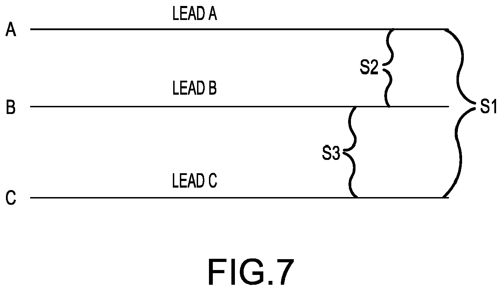

In FIG. 5, the process starts at S520 with detecting first signals, second signals and third signals at a controller via three leads connected to the first passive ultrasound sensor, the second passive ultrasound sensor and the third passive ultrasound sensor. The configuration of three passive ultrasound sensors with three leads is shown in FIG. 7 and explained with reference to FIG. 7. The detecting at S520 may be performed by the controller 250.

At S525, the process in FIG. 5 includes automatically detecting when the first signals, the second signals, and the third signals are simultaneously received. In this regard, simultaneous receipt of the three signals may reflect a problem such as that the first passive ultrasound sensor S1, the second passive ultrasound sensor S2 and the third passive ultrasound sensor S3 are aligned linearly from the viewpoint of the imaging frame at the center of the imaging beam from the ultrasound imaging probe 210. As such, the three passive ultrasound sensors may not be differentiable based on timing, though this can be remedied by simply adjusting the ultrasound imaging probe 210 to fire another imaging beam from another position. The detecting at S525 may be performed by the controller 250.

At S530, the process in FIG. 5 includes re-performing the process until the first signals, the second signals and the third signals are not simultaneously received.

At S540, the process in FIG. 5 includes identifying a characteristic of the first signals, the second signals and the third signals at the controller. The characteristic includes at least one of shapes of the first signals, the second signals and the third signals, and times at which the first signals, the second signals and the third signals are generated. The identifying may be performed by the controller 250.

At S580, the process in FIG. 5 includes differentiating between the first signals, the second signals and the third signals at the controller. The differentiating at S580 is based on the characteristic including the at least one of the shapes of the first signals, the second signals and the third signals, and the times at which the first signals, the second signals and the third signals are generated. The differentiating at S580 may be performed by the controller 250.

At S590, the process in FIG. 5 includes displaying and tracking the first passive ultrasound sensor, the second passive ultrasound sensor and the third passive ultrasound sensor. The displaying and tracking at S590 is also based on the characteristic including the at least one of the shapes of the first signals, the second signals and the third signals and the times at which the first signals, the second signals and the third signals are generated. The displaying at S590 may be performed by the monitor 295, whereas the tracking may be performed by the combination of the controller 250 and the monitor 295. For example, the controller 250 may identify locations of the three passive ultrasound sensors and provide the locations for display with ultrasound imagery on the monitor 295. The differentiation can be used to label each of the three passive ultrasound sensors, which in turn may be used to identify a pose of the interventional medical device insofar as the arrangement of the three passive ultrasound sensors on the interventional medical device may be known in advance.

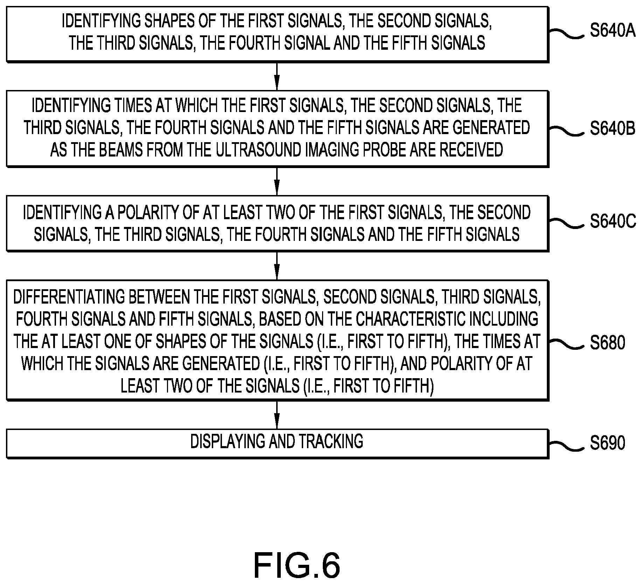

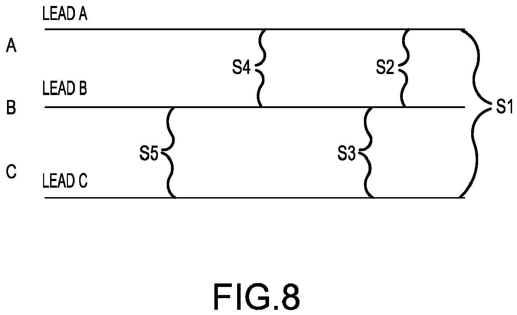

FIG. 6 illustrates another process for differentiating passive ultrasound sensors for interventional medical procedures, in accordance with a representative embodiment. In FIG. 6, the process starts at S640A by identifying shapes of the first signals, the second signals, the third signals, the fourth signals and the fifth signals. The process of FIG. 6 may correspond to a configuration with five passive ultrasound sensors with three leads as is shown in FIG. 8 and explained with reference to FIG. 8. The shapes of the signals identified at S640A may be individual parameters such as lengths of the signals. The identifying at S640A may be performed by a controller 250.

At S640B, the process in FIG. 6 includes identifying times at which the first signals, the second signals, the third signals, the fourth signals and the fifth signals are generated as the beams from the ultrasound imaging probe are received. The times at which the signals are generated at S640B may correspond to when different bias voltages are applied to amplifiers in a predetermined pattern, so that whichever signals are generated at certain times may be limited to one or two of the passive ultrasound sensors. The identifying at S640B may be performed by a controller 250.

At S640C, the process in FIG. 6 includes identifying a polarity of at least two of the first signals, the second signals, the third signals, the fourth signals and the fifth signals. The polarity may simply be whether the peak voltage reading of any or all of the five signals is positive or negative. A polarity characteristic of signals can be identified by a process implemented by the controller 250. The identifying at S640C may be performed by a controller 250.

At S680, the process in FIG. 6 includes differentiating between the first signals, the second signals, the third signals, the fourth signals and the fifth signals. The differentiating at S680 is based on the characteristic including the at least one of shapes of the signals (i.e., the first to fifth signals), the times at which the signals are generated (i.e., the first to fifth signals), and the polarity of at least two of the signals (i.e., the first to fifth signals). For example, when only one signal is received it may reflect a certain bias voltage being applied to the amplifiers as explained above, and when two signals are received it may reflect a different bias voltage. The same is true for signals with the opposite polarity. The differentiating at S680 may be performed by a controller.

At S690, the process in FIG. 6 includes displaying and tracking the first passive ultrasound sensor, the second passive ultrasound sensor, the third passive ultrasound sensor, the fourth passive ultrasound sensor and the fifth passive ultrasound sensor. The displaying and tracking at S690 is based on the characteristic including the at least one of shapes of the first signals, the second signals, the third signals, the fourth signals and the fifth signals, and the times at which the first signals, the second signals, the third signals, the fourth signals and the fifth signals are generated.

FIG. 7 illustrates a set of passive ultrasound sensors with differentiating connections in a single configuration for differentiating passive ultrasound sensors for interventional medical procedures, in accordance with a representative embodiment.

In the embodiment of FIG. 7, three passive ultrasound sensors are connected between three leads. The three leads in FIG. 7 may be connected between the passive ultrasound sensors and a controller 250. The three passive ultrasound sensors include the first passive ultrasound sensor S1, the second passive ultrasound sensor S2 and the third passive ultrasound sensor S3. The first passive ultrasound sensor S1 is connected between lead A and lead C. The second passive ultrasound sensor S2 is connected between lead A and lead B. The third passive ultrasound sensor S3 is connected between lead B and lead C.

In the embodiment of FIG. 7, it is possible to detect which passive ultrasound sensor is generating a signal, as long as no sensors are insonified simultaneously. If a signal is observed between lead A and lead C, the signal is from the first passive ultrasound sensor S1. If the signal is observed between lead A and lead B, the signal is from the second passive ultrasound sensor S2. If the signal is observed between lead B and lead C, the signal is from the third passive ultrasound sensor S3.

FIG. 8 illustrates another set of passive ultrasound sensors with differentiating connections in a single configuration for differentiating passive ultrasound sensors for interventional medical procedures, in accordance with a representative embodiment.

In the embodiment of FIG. 8, five passive ultrasound sensors are connected between three leads. The three leads in FIG. 8 may be connected between the passive ultrasound sensors and a controller 250. The five passive ultrasound sensors include the first passive ultrasound sensor S1, the second passive ultrasound sensor S2, the third passive ultrasound sensor S3, the fourth passive ultrasound sensor S4 and the fifth passive ultrasound sensor S5. The first passive ultrasound sensor S1 is connected between lead A and lead C. The second passive ultrasound sensor S2 is connected between lead A and lead B. The third passive ultrasound sensor S3 is connected between lead B and lead C. The fourth passive ultrasound sensor S4 is connected between lead A and lead B. The fifth passive ultrasound sensor S5 is connected between lead B and lead C.

The embodiment of FIG. 8 expands on the embodiment of FIG. 7. In FIG. 8, one or more pairs of the passive ultrasound sensors may be connected back-to-back with different-polarities. Insofar as an acoustic emission of an ultrasound imaging probe is generally non-symmetric and can, for example, have a higher peak positive pressure than peak negative pressure, when connecting two passive ultrasound sensors in parallel, this asymmetry can be exploited by inverting the polarity of one passive ultrasound sensor with respect to the other.

For example, the second passive ultrasound sensor S2 and the fourth passive ultrasound sensor S4 may be connected in parallel at different locations on the device but to the same pair of wires. In this example, the fourth passive ultrasound sensor S4 may be connected in reverse relative to the second passive ultrasound sensor S2, so that the polarity is reversed for the fourth passive ultrasound sensor S4. As the second passive ultrasound sensor S2 and the fourth passive ultrasound sensor S4 are in different locations, they will be hit by the imaging beam at different times. When a strong signal is received from the wire pair, the identity of the second passive ultrasound sensor S2 and the fourth passive ultrasound sensor S4 can be deduced based on the signals from the second passive ultrasound sensor S2 and the fourth passive ultrasound sensor S4. A signal with a maximum positive signal bigger than the maximum negative signal will be from the second passive ultrasound sensor S2, and the signal with a maximum negative signal bigger than the maximum positive signal will be from the fourth passive ultrasound sensor S4.

In FIG. 8, the first passive ultrasound sensor S1 is at the tip of the interventional medical device and is the only passive ultrasound sensor connected directly between leads A and C. The signal from the first passive ultrasound sensor S1 can be optimally detected for the tip tracking. Leads A and C may be two wires running inside a catheter braided conductive mesh which is a structural part of the catheter. The braided mesh may act as a shield to improve signal-to-noise rations for the first passive ultrasound sensor S1 but also as a signal lead when receiving signals on the other four passive ultrasound sensors.

The second passive ultrasound sensor S2 and the fourth passive ultrasound sensor S4 are connected in parallel between leads A and B, with the fourth passive ultrasound sensor S4 having reversed polarity compared to the second passive ultrasound sensor S2. Similarly, the third passive ultrasound sensor S3 and the fifth passive ultrasound sensor S5 are connected between leads B and C with the polarity of the fifth passive ultrasound sensor S5 reversed relative to the polarity of the third passive ultrasound sensor S3. The configuration of FIG. 8 creates an optimal signal-to-noise ratio for the first passive ultrasound sensor S1 at the tip so that it can be accurately tracked, while also allowing detection of the four remaining passive ultrasound sensors at lower signal-to-noise ratios insofar as the braid structure will pick up more noise. The lower signal-to-noise ration on the four remaining sensors is less problematic when used for orientation determination of the interventional medical device. For example, a needle with the five passive ultrasound sensors shown in FIG. 8 may have the second passive ultrasound sensor S2, the third passive ultrasound sensor S3, the fourth passive ultrasound sensor S4 and the fifth passive ultrasound sensor S5 placed along the needle shaft and be closer to the ultrasound imaging probe then the first passive ultrasound sensor S1 at the tip and thus not require as much sensitivity for the second passive ultrasound sensor S2 to the fifth passive ultrasound sensor S5.

Accordingly, differentiating passive ultrasound sensors for interventional medical procedures enables integration of multiple passive ultrasound sensors in or on an interventional medical device to track orientation and bending/deployment of the interventional medical device. The multiple passive ultrasound sensors can be integrated at low cost and with a minimized number of (e.g., shared) electrical leads used to connect the passive ultrasound sensors. The sensor differentiation described herein may be implemented by modifying existing passive ultrasound sensors or manufacturing new passive ultrasound sensors such that they each have unique detectable behavior. In the event that the passive ultrasound sensors are newly manufactured, such passive ultrasound sensors can be mass manufactured without overly complicating the manufacturing process while still enabling the differentiation described herein.

As set forth above, shapes of received signals such as the length of received signals can be used as a characteristic to differentiate passive ultrasound sensors. Similarly, timing of signals such as when signal are generated (and implicitly when they are not generated) may be used as a characteristic to differentiate passive ultrasound sensors. Additionally, polarity of signals can be used to differentiate passive ultrasound sensors. These three types of characteristics can be used in combination to differentiate between multiple passive ultrasound sensors.

Although differentiating passive ultrasound sensors for interventional medical procedures has been described with reference to several exemplary embodiments, it is understood that the words that have been used are words of description and illustration, rather than words of limitation. Changes may be made within the purview of the appended claims, as presently stated and as amended, without departing from the scope and spirit of differentiating passive ultrasound sensors for interventional medical procedures in its aspects. Although differentiating passive ultrasound sensors for interventional medical procedures has been described with reference to particular means, materials and embodiments, differentiating passive ultrasound sensors for interventional medical procedures is not intended to be limited to the particulars disclosed; rather differentiating passive ultrasound sensors for interventional medical procedures extends to all functionally equivalent structures, methods, and uses such as are within the scope of the appended claims.

The following Examples are provided:
Example 1. A controller (250) for differentiating passive ultrasound sensors (S1, S2) for interventional medical procedures, comprising:
   a memory (291) that stores instructions, and
   a processor (292) that executes the instructions, wherein, when executed by the processor (292), the instructions cause a system (200) that includes the controller (250) to implement a process that includes:
      receiving (S320) first signals from a first passive ultrasound sensor (S1) that generates the first signals responsive to beams emitted from an ultrasound imaging probe (210);
      receiving (S320) second signals from a second passive ultrasound sensor (S2) that generates the second signals responsive to the beams emitted from the ultrasound imaging probe (210);
      identifying (S340) a characteristic of the first signals and the second signals including at least one of shapes of the first signals and the second signals and times at which the first signals and the second signals are generated as the beams from the ultrasound imaging probe (210) are received; and
      differentiating (S380) between the first passive ultrasound sensor (S1) and the second passive ultrasound sensor (S2) based on the characteristic including the at least one of shapes of the first signals and the second signals and the times at which the first signals and the second signals are generated as the beams from the ultrasound imaging probe (210) are received.
Example 2. The controller (250) of Example 1,
   wherein the characteristic of the first signals and the second signals comprises shapes of the first signals and the second signals, and the shapes of the first signals and the second signals reflect at least one of sizes of the first passive ultrasound sensor (S1) and the second passive ultrasound sensor (S2), and time durations during which the first passive ultrasound sensor (S1) and the second passive ultrasound sensor (S2) received the beams emitted from the ultrasound imaging probe (210).
Example 3. The controller (250) of Example 1,
   wherein the process implemented when the controller (250) executes the instructions further comprises: controlling an amplifier voltage from an amplifier to bias the first passive ultrasound sensor (S1) and the second passive ultrasound sensor (S2) with varied bias voltages so that only the first passive ultrasound sensor (S1) generates the first signals when the amplifier produces a first bias voltage, and so that both the first passive ultrasound sensor (S1) generates the first signals and the second passive ultrasound sensor (S2) generates the second signals when the amplifier voltage produces a second bias voltage, and
   wherein the characteristic of the first signals and the second signals comprises times at which the first signals and the second signals are generated as the beams from the ultrasound imaging probe (210) are received, and the times at which the first signals and the second signals are generated as the beams from the ultrasound imaging probe (210) are received are differentiated based on when the amplifier produces the first bias voltage and when the amplifier produces the second bias voltage.
Example 4. The controller (250) of Example 1,
   wherein the process implemented when the controller (250) executes the instructions further comprises:
   receiving (S320) third signals from a third passive ultrasound sensor (S3) that generates the third signals responsive to the beams emitted from the ultrasound imaging probe (210);
   identifying (S340) the characteristic of the third signals, including at least one of shapes of the third signals and the times at which the third signals are generated as the beams from the ultrasound imaging probe (210) are received; and
   differentiating (S380) between the first passive ultrasound sensor (S1), the second passive ultrasound sensor (S2) and the third passive ultrasound sensor (S3) based on the characteristic.
Example 5. The controller (250) of Example 4,
   wherein the process implemented when the controller (250) executes the instructions further comprises: detecting (FIG. 7) the first signals, the second signals and the third signals from three leads connected to the first passive ultrasound sensor (S1), the second passive ultrasound sensor (S2) and the third passive ultrasound sensor (S3), and
   wherein each of the first passive ultrasound sensor (S1), the second passive ultrasound sensor (S2) and the third passive ultrasound sensor (S3) is connected between a different two of the three leads.
Example 6. The controller (250) of Example 5,
   wherein the process implemented when the controller (250) executes the instructions further comprises: automatically detecting when the first signals, the second signals and the third signals are simultaneously received, and re-performing the process until the first signals, the second signals and the third signals are not simultaneously received.
Example 7. The controller (250) of Example 4,
   wherein the process implemented when the controller (250) executes the instructions further comprises:
   receiving (S320) fourth signals from a fourth passive ultrasound sensor (S4) that generates the fourth signals responsive to the beams emitted from the ultrasound imaging probe (210);
   receiving (S320) fifth signals from a fifth passive ultrasound sensor (S5) that generates the fifth signals responsive to the beams emitted from the ultrasound imaging probe (210);
   identifying (S340) the characteristic of the fourth signals and the fifth signals, including at least one of shapes of the fourth signals and the fifth signals and the times at which the fourth signals and the fifth signals are generated as the beams from the ultrasound imaging probe (210) are received; and
   differentiating (S380) between the first passive ultrasound sensor (S1), the second passive ultrasound sensor (S2), the third passive ultrasound sensor (S3), the fourth passive ultrasound sensor (S4) and the fifth passive ultrasound sensor (S5) based on the characteristic.
Example 8. The controller (250) of Example 7,
   wherein the process implemented when the controller (250) executes the instructions further comprises:
   identifying (S640C) a polarity characteristic of two of the first signals, the second signals, the third signals, the fourth signals and the fifth signals, and
   differentiating (S680) between two of the first passive ultrasound sensor (S1), the second passive ultrasound sensor (S2), the third passive ultrasound sensor (S3), the fourth passive ultrasound sensor (S4) and the fifth passive ultrasound sensor (S5) based on the polarity characteristic.
Example 9. A tangible non-transitory computer readable storage medium (291) that stores a computer program, the computer program, when executed by a processor (292), causing a system (200) that includes the tangible non-transitory computer readable storage medium (291) to perform a process for differentiating passive ultrasound sensors for interventional medical procedures, the process performed when the processor (292) executes the computer program comprising:
   receiving (S320) first signals from a first passive ultrasound sensor (S1) that generates the first signals responsive to beams emitted from an ultrasound imaging probe (210);
   receiving (S320) second signals from a second passive ultrasound sensor (S2) that generates the second signals responsive to the beams emitted from the ultrasound imaging probe (210);
   identifying (S340) a characteristic of the first signals and the second signals including at least one of shapes of the first signals and the second signals and times at which the first signals and the second signals are generated as the beams from the ultrasound imaging probe (210) are received; and
   differentiating (S380) between the first passive ultrasound sensor (S1) and the second passive ultrasound sensor (S2) based on the characteristic including the at least one of shapes of the first signals and the second signals and the times at which the first signals and the second signals are generated as the beams from the ultrasound imaging probe (210) are received.
Example 10. The tangible non-transitory computer readable storage medium (291) of Example 9,
   wherein the characteristic of the first signals and the second signals comprises shapes of the first signals and the second signals, and the shapes of the first signals and the second signals reflect at least one of sizes of the first passive ultrasound sensor (S1) and the second passive ultrasound sensor (S2), and time durations during which the first passive ultrasound sensor (S1) and the second passive ultrasound sensor (S2) received the beams emitted from the ultrasound imaging probe (210).
Example 11. The tangible non-transitory computer readable storage medium (291) of Example 9, wherein the process implemented by the system (200) further comprises:
   controlling an amplifier voltage from an amplifier to bias the first passive ultrasound sensor (S1) and the second passive ultrasound sensor (S2) with varied bias voltages so that only the first passive ultrasound sensor (S1) generates the first signals when the amplifier produces a first bias voltage, and so that both the first passive ultrasound sensor (S1) generates the first signals and the second passive ultrasound sensor (S2) generates the second signals when the amplifier voltage produces a second bias voltage, and
   wherein the characteristic of the first signals and the second signals comprises times at which the first signals and the second signals are generated as the beams from the ultrasound imaging probe (210) are received, and the times at which the first signals and the second signals are generated as the beams from the ultrasound imaging probe (210) are received are differentiated based on when the amplifier produces the first bias voltage and when the amplifier produces the second bias voltage.
Example 12. The tangible non-transitory computer readable storage medium (291) of Example 9, wherein the process implemented by the system (200) further comprises:
   receiving (S320) third signals from a third passive ultrasound sensor (S3) that generates the third signals responsive to the beams emitted from the ultrasound imaging probe (210);
   identifying (S340) the characteristic of the third signals, including at least one of shapes of the third signals and the times at which the third signals are generated as the beams from the ultrasound imaging probe (210) are received; and
   differentiating (S380) between the first passive ultrasound sensor (S1), the second passive ultrasound sensor (S2) and the third passive ultrasound sensor (S3) based on the characteristic.
Example 13. The tangible non-transitory computer readable storage medium (291) of Example 12, wherein the process implemented by the system (200) further comprises:
   detecting (FIG. 7) the first signals, the second signals and the third signals from three leads connected to the first passive ultrasound sensor (S1), the second passive ultrasound sensor (S2) and the third passive ultrasound sensor (S3), and
   wherein each of the first passive ultrasound sensor (S1), the second passive ultrasound sensor (S2) and the third passive ultrasound sensor (S3) is connected between a different two of the three leads.
Example 14. The tangible non-transitory computer readable storage medium (291) of Example 13, wherein the process implemented by the system (200) further comprises:
   automatically detecting when the first signals, the second signals and the third signals are simultaneously received, and re-performing the process until the first signals, the second signals and the third signals are not simultaneously received.
Example 15. The tangible non-transitory computer readable storage medium (291) of Example 12, wherein the process implemented by the system (200) further comprises:
   receiving (S320) fourth signals from a fourth passive ultrasound sensor (S4) that generates the fourth signals responsive to the beams emitted from the ultrasound imaging probe (210);
   receiving (S320) fifth signals from a fifth passive ultrasound sensor (S5) that generates the fifth signals responsive to the beams emitted from the ultrasound imaging probe (210);
   identifying (S340) the characteristic of the fourth signals and the fifth signals, including at least one of shapes of the fourth signals and the fifth signals and the times at which the fourth signals and the fifth signals are generated as the beams from the ultrasound imaging probe (210) are received; and
   differentiating (S380) between the first passive ultrasound sensor (S1), the second passive ultrasound sensor (S2), the third passive ultrasound sensor (S3), the fourth passive ultrasound sensor (S4) and the fifth passive ultrasound sensor (S5) based on the characteristic.
Example 16. The tangible non-transitory computer readable storage medium (291) of Example 15, wherein the process implemented by the system (200) further comprises:
   identifying (S640C) a polarity characteristic of two of the first signals, the second signals, the third signals, the fourth signals and the fifth signals, and
   differentiating (S680) between two of the first passive ultrasound sensor (S1), the second passive ultrasound sensor (S2), the third passive ultrasound sensor (S3), the fourth passive ultrasound sensor (S4) and the fifth passive ultrasound sensor (S5) based on the polarity characteristic.
Example 17. A system (200) for differentiating passive ultrasound sensors for an interventional medical procedure, comprising:
   a first passive ultrasound sensor (S1) that generates and sends first signals responsive to beams emitted from an ultrasound imaging probe (210) during an interventional medical procedure;
   a second passive ultrasound sensor (S2) that generates and sends second signals responsive to the beams emitted from the ultrasound imaging probe (210);
   wherein the first signals and the second signals include an identifiable characteristic including at least one of shapes of the first signals and the second signals and times at which the first signals and the second signals are generated as the beams from the ultrasound imaging probe (210) are received, so that the first passive ultrasound sensor (S1) and the second passive ultrasound sensor (S2) can be differentiated based on the identifiable characteristic including the at least one of shapes of the first signals and the second signals and the times at which the first signals and the second signals are generated as the beams from the ultrasound imaging probe (210) are received.
Example 18. The system (200) of Example 17, further comprising:
   the ultrasound imaging probe (210) that emits beams during the interventional medical procedure; and
   a controller (250) comprising a memory (291) that stores instructions and a processor (292) that executes the instructions, wherein, when executed by the processor (292), the instructions cause the system (200) to implement a process that includes:
      receiving (S320) the first signals from the first passive ultrasound sensor (S1);
      receiving (S320) the second signals from the second passive ultrasound sensor (S2);
      identifying (S340) the identifiable characteristic of the first signals and the second signals; and
      differentiating (S380) between the first passive ultrasound sensor (S1) and the second passive ultrasound sensor (S2) based on the identifiable characteristic.
Example 19. The system (200) of Example 18, further comprising:
   a third passive ultrasound sensor (S3) that generates and sends third signals responsive to beams emitted from an ultrasound imaging probe (210) during the interventional medical procedure,
   wherein the process implemented when the controller (250) executes the instructions further comprises:
      receiving (S320) the third signals from a third passive ultrasound sensor (S3);
      identifying (S340) the identifiable characteristic of the third signals; and
      differentiating (S380) between the first passive ultrasound sensor (S1), the second passive ultrasound sensor (S2) and the third passive ultrasound sensor (S3) based on the identifiable characteristic.
Example 20. The system (200) of Example 19, further comprising:
   a fourth passive ultrasound sensor (S4) that generates and sends fourth signals responsive to beams emitted from an ultrasound imaging probe (210) during the interventional medical procedure,
   a fifth passive ultrasound sensor (S5) that generates and sends fifth signals responsive to beams emitted from an ultrasound imaging probe (210) during the interventional medical procedure,
   wherein the process implemented when the controller (250) executes the instructions further comprises:
      receiving (S320) the fourth signals from the fourth passive ultrasound sensor (S4);
      receiving (S320) the fifth signals from the fifth passive ultrasound sensor (S5);
      identifying (S340) the identifiable characteristic of the fourth signals and the fifth signals; and
      differentiating (S380) between the first passive ultrasound sensor (S1), the second passive ultrasound sensor (S2), the third passive ultrasound sensor (S3), the fourth passive ultrasound sensor (S4) and the fifth passive ultrasound sensor (S5) based on the identifiable characteristic.

The illustrations of the embodiments described herein are intended to provide a general understanding of the structure of the various embodiments. The illustrations are not intended to serve as a complete description of all of the elements and features of the disclosure described herein. Many other embodiments may be apparent to those of skill in the art upon reviewing the disclosure. Other embodiments may be utilized and derived from the disclosure, such that structural and logical substitutions and changes may be made without departing from the scope of the disclosure. Additionally, the illustrations are merely representational and may not be drawn to scale. Certain proportions within the illustrations may be exaggerated, while other proportions may be minimized. Accordingly, the disclosure and the figures are to be regarded as illustrative rather than restrictive.

One or more embodiments of the disclosure may be referred to herein, individually and/or collectively, by the term "invention" merely for convenience and without intending to voluntarily limit the scope of this application to any particular invention or inventive concept. Moreover, although specific embodiments have been illustrated and described herein, it should be appreciated that any subsequent arrangement designed to achieve the same or similar purpose may be substituted for the specific embodiments shown. This disclosure is intended to cover any and all subsequent adaptations or variations of various embodiments. Combinations of the above embodiments, and other embodiments not specifically described herein, will be apparent to those of skill in the art upon reviewing the description.

The Abstract of the Disclosure is provided to comply with 37 C.F.R. § 1.72(b) and is submitted with the understanding that it will not be used to interpret or limit the scope or meaning of the claims. In addition, in the foregoing Detailed Description, various features may be grouped together or described in a single embodiment for the purpose of streamlining the disclosure. This disclosure is not to be interpreted as reflecting an intention that the claimed embodiments require more features than are expressly recited in each claim. Rather, as the following claims reflect, inventive subject matter may be directed to less than all of the features of any of the disclosed embodiments. Thus, the following claims are incorporated into the Detailed Description, with each claim standing on its own as defining separately claimed subject matter.

The preceding description of the disclosed embodiments is provided to enable any person skilled in the art to practice the concepts described in the present disclosure. As such, the above disclosed subject matter is to be considered illustrative, and not restrictive, and the appended claims are intended to cover all such modifications, enhancements, and other embodiments which fall within the true spirit and scope of the present disclosure. Thus, to the maximum extent allowed by law, the scope of the present disclosure is to be determined by the broadest permissible interpretation of the following claims and their equivalents and shall not be restricted or limited by the foregoing detailed description.

## Claims

1. A controller (250) for differentiating passive ultrasound sensors (S1, S2) for interventional medical procedures, comprising:
a memory (291) that stores instructions, and
a processor (292) that executes the instructions, wherein, when executed by the processor (292), the instructions cause a system (200) that includes the controller (250) to implement a process that includes:
receiving (S320) first signals from a first passive ultrasound sensor (S1) that generates the first signals responsive to beams emitted from an ultrasound imaging probe (210);
receiving (S320) second signals from a second passive ultrasound sensor (S2) that generates the second signals responsive to the beams emitted from the ultrasound imaging probe (210);
identifying (S340) a characteristic of the first signals and the second signals including at least one of shapes of the first signals and the second signals and times at which the first signals and the second signals are generated as the beams from the ultrasound imaging probe (210) are received; and
differentiating (S380) between the first passive ultrasound sensor (S1) and the second passive ultrasound sensor (S2) based on the characteristic including the at least one of shapes of the first signals and the second signals and the times at which the first signals and the second signals are generated as the beams from the ultrasound imaging probe (210) are received.

2. The controller (250) of claim 1,
wherein the characteristic of the first signals and the second signals comprises shapes of the first signals and the second signals, and the shapes of the first signals and the second signals reflect at least one of sizes of the first passive ultrasound sensor (S1) and the second passive ultrasound sensor (S2), and time durations during which the first passive ultrasound sensor (S1) and the second passive ultrasound sensor (S2) received the beams emitted from the ultrasound imaging probe (210).

3. The controller (250) of claim 1,
wherein the process implemented when the controller (250) executes the instructions further comprises: controlling an amplifier voltage from an amplifier to bias the first passive ultrasound sensor (S1) and the second passive ultrasound sensor (S2) with varied bias voltages so that only the first passive ultrasound sensor (S1) generates the first signals when the amplifier produces a first bias voltage, and so that both the first passive ultrasound sensor (S1) generates the first signals and the second passive ultrasound sensor (S2) generates the second signals when the amplifier voltage produces a second bias voltage, and
wherein the characteristic of the first signals and the second signals comprises times at which the first signals and the second signals are generated as the beams from the ultrasound imaging probe (210) are received, and the times at which the first signals and the second signals are generated as the beams from the ultrasound imaging probe (210) are received are differentiated based on when the amplifier produces the first bias voltage and when the amplifier produces the second bias voltage.

4. The controller (250) of claim 1,
wherein the process implemented when the controller (250) executes the instructions further comprises:
receiving (S320) third signals from a third passive ultrasound sensor (S3) that generates the third signals responsive to the beams emitted from the ultrasound imaging probe (210);
identifying (S340) the characteristic of the third signals, including at least one of shapes of the third signals and the times at which the third signals are generated as the beams from the ultrasound imaging probe (210) are received; and
differentiating (S380) between the first passive ultrasound sensor (S1), the second passive ultrasound sensor (S2) and the third passive ultrasound sensor (S3) based on the characteristic.

5. The controller (250) of claim 4,
wherein the process implemented when the controller (250) executes the instructions further comprises: detecting (FIG. 7) the first signals, the second signals and the third signals from three leads connected to the first passive ultrasound sensor (S1), the second passive ultrasound sensor (S2) and the third passive ultrasound sensor (S3), and
wherein each of the first passive ultrasound sensor (S1), the second passive ultrasound sensor (S2) and the third passive ultrasound sensor (S3) is connected between a different two of the three leads.

6. The controller (250) of claim 5,
wherein the process implemented when the controller (250) executes the instructions further comprises: automatically detecting when the first signals, the second signals and the third signals are simultaneously received, and re-performing the process until the first signals, the second signals and the third signals are not simultaneously received.

7. The controller (250) of claim 4,
wherein the process implemented when the controller (250) executes the instructions further comprises:
receiving (S320) fourth signals from a fourth passive ultrasound sensor (S4) that generates the fourth signals responsive to the beams emitted from the ultrasound imaging probe (210);
receiving (S320) fifth signals from a fifth passive ultrasound sensor (S5) that generates the fifth signals responsive to the beams emitted from the ultrasound imaging probe (210);
identifying (S340) the characteristic of the fourth signals and the fifth signals, including at least one of shapes of the fourth signals and the fifth signals and the times at which the fourth signals and the fifth signals are generated as the beams from the ultrasound imaging probe (210) are received; and
differentiating (S380) between the first passive ultrasound sensor (S1), the second passive ultrasound sensor (S2), the third passive ultrasound sensor (S3), the fourth passive ultrasound sensor (S4) and the fifth passive ultrasound sensor (S5) based on the characteristic.

8. The controller (250) of claim 7,
wherein the process implemented when the controller (250) executes the instructions further comprises:
identifying (S640C) a polarity characteristic of two of the first signals, the second signals, the third signals, the fourth signals and the fifth signals, and
differentiating (S680) between two of the first passive ultrasound sensor (S1), the second passive ultrasound sensor (S2), the third passive ultrasound sensor (S3), the fourth passive ultrasound sensor (S4) and the fifth passive ultrasound sensor (S5) based on the polarity characteristic.

9. A tangible non-transitory computer readable storage medium (291) that stores a computer program, the computer program, when executed by a processor (292), causing a system (200) that includes the tangible non-transitory computer readable storage medium (291) to perform a process for differentiating passive ultrasound sensors for interventional medical procedures, the process performed when the processor (292) executes the computer program comprising:
receiving (S320) first signals from a first passive ultrasound sensor (S1) that generates the first signals responsive to beams emitted from an ultrasound imaging probe (210);
receiving (S320) second signals from a second passive ultrasound sensor (S2) that generates the second signals responsive to the beams emitted from the ultrasound imaging probe (210);
identifying (S340) a characteristic of the first signals and the second signals including at least one of shapes of the first signals and the second signals and times at which the first signals and the second signals are generated as the beams from the ultrasound imaging probe (210) are received; and
differentiating (S380) between the first passive ultrasound sensor (S1) and the second passive ultrasound sensor (S2) based on the characteristic including the at least one of shapes of the first signals and the second signals and the times at which the first signals and the second signals are generated as the beams from the ultrasound imaging probe (210) are received.

10. The tangible non-transitory computer readable storage medium (291) of claim 9,
wherein the characteristic of the first signals and the second signals comprises shapes of the first signals and the second signals, and the shapes of the first signals and the second signals reflect at least one of sizes of the first passive ultrasound sensor (S1) and the second passive ultrasound sensor (S2), and time durations during which the first passive ultrasound sensor (S1) and the second passive ultrasound sensor (S2) received the beams emitted from the ultrasound imaging probe (210).

11. The tangible non-transitory computer readable storage medium (291) of claim 9, wherein the process implemented by the system (200) further comprises:
controlling an amplifier voltage from an amplifier to bias the first passive ultrasound sensor (S1) and the second passive ultrasound sensor (S2) with varied bias voltages so that only the first passive ultrasound sensor (S1) generates the first signals when the amplifier produces a first bias voltage, and so that both the first passive ultrasound sensor (S1) generates the first signals and the second passive ultrasound sensor (S2) generates the second signals when the amplifier voltage produces a second bias voltage, and
wherein the characteristic of the first signals and the second signals comprises times at which the first signals and the second signals are generated as the beams from the ultrasound imaging probe (210) are received, and the times at which the first signals and the second signals are generated as the beams from the ultrasound imaging probe (210) are received are differentiated based on when the amplifier produces the first bias voltage and when the amplifier produces the second bias voltage.

12. A system (200) for differentiating passive ultrasound sensors for an interventional medical procedure, comprising:
a first passive ultrasound sensor (S1) that generates and sends first signals responsive to beams emitted from an ultrasound imaging probe (210) during an interventional medical procedure;
a second passive ultrasound sensor (S2) that generates and sends second signals responsive to the beams emitted from the ultrasound imaging probe (210);
wherein the first signals and the second signals include an identifiable characteristic including at least one of shapes of the first signals and the second signals and times at which the first signals and the second signals are generated as the beams from the ultrasound imaging probe (210) are received, so that the first passive ultrasound sensor (S1) and the second passive ultrasound sensor (S2) can be differentiated based on the identifiable characteristic including the at least one of shapes of the first signals and the second signals and the times at which the first signals and the second signals are generated as the beams from the ultrasound imaging probe (210) are received.

13. The system (200) of claim 12, further comprising:
the ultrasound imaging probe (210) that emits beams during the interventional medical procedure; and
a controller (250) comprising a memory (291) that stores instructions and a processor (292) that executes the instructions, wherein, when executed by the processor (292), the instructions cause the system (200) to implement a process that includes:
receiving (S320) the first signals from the first passive ultrasound sensor (S1);
receiving (S320) the second signals from the second passive ultrasound sensor (S2);
identifying (S340) the identifiable characteristic of the first signals and the second signals; and
differentiating (S380) between the first passive ultrasound sensor (S1) and the second passive ultrasound sensor (S2) based on the identifiable characteristic.

14. The system (200) of claim 13, further comprising:
a third passive ultrasound sensor (S3) that generates and sends third signals responsive to beams emitted from an ultrasound imaging probe (210) during the interventional medical procedure,
wherein the process implemented when the controller (250) executes the instructions further comprises:
receiving (S320) the third signals from a third passive ultrasound sensor (S3);
identifying (S340) the identifiable characteristic of the third signals; and
differentiating (S380) between the first passive ultrasound sensor (S1), the second passive ultrasound sensor (S2) and the third passive ultrasound sensor (S3) based on the identifiable characteristic.

15. The system (200) of claim 14, further comprising:
a fourth passive ultrasound sensor (S4) that generates and sends fourth signals responsive to beams emitted from an ultrasound imaging probe (210) during the interventional medical procedure,
a fifth passive ultrasound sensor (S5) that generates and sends fifth signals responsive to beams emitted from an ultrasound imaging probe (210) during the interventional medical procedure,
wherein the process implemented when the controller (250) executes the instructions further comprises:
receiving (S320) the fourth signals from the fourth passive ultrasound sensor (S4);
receiving (S320) the fifth signals from the fifth passive ultrasound sensor (S5);
identifying (S340) the identifiable characteristic of the fourth signals and the fifth signals; and
differentiating (S380) between the first passive ultrasound sensor (S1), the second passive ultrasound sensor (S2), the third passive ultrasound sensor (S3), the fourth passive ultrasound sensor (S4) and the fifth passive ultrasound sensor (S5) based on the identifiable characteristic.
